(19) Europäisches Patentamt
European Patent Office
Office européen des brevets

(11) **EP 2 659 882 A1**

(12) **EUROPEAN PATENT APPLICATION**
published in accordance with Art. 153(4) EPC

(43) Date of publication:
06.11.2013 Bulletin 2013/45

(51) Int Cl.:
*A61K 9/14* (2006.01)   *A61K 47/02* (2006.01)
*A61K 47/04* (2006.01)   *A61K 47/26* (2006.01)
*A61K 47/32* (2006.01)   *A61K 47/36* (2006.01)
*A61K 47/38* (2006.01)

(21) Application number: 11854059.0

(22) Date of filing: 27.12.2011

(86) International application number:
PCT/JP2011/080276

(87) International publication number:
WO 2012/091040 (05.07.2012 Gazette 2012/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 27.12.2010 JP 2010290831

(71) Applicant: Tomita Pharmaceutical Co., Ltd.
Naruto-shi
Tokushima 771-0360 (JP)

(72) Inventors:
• KONISHI, Masashi
Naruto-shi, Tokushima 771-0360 (JP)
• KAWAMOTO, Ariumi
Naruto-shi, Tokushima 771-0360 (JP)
• OOKUBO, Akira
Naruto-shi, Tokushima 771-0360 (JP)
• YUNOKI, Masashi
Naruto-shi, Tokushima 771-0360 (JP)

(74) Representative: Müller-Boré & Partner
Patentanwälte
Grafinger Straße 2
81671 München (DE)

(54) **DECAY-TYPE NUCLEAR PARTICLE FOR PHARMACEUTICAL FORMULATION**

(57)     Provided is a core particle for a pharmaceutical preparation, capable of delivering a relatively large amount of a drug active ingredient, in particular, at a predetermined timing. This invention relates to a disintegratable core particle for a pharmaceutical preparation, which core particle is adapted for formation, on the particle surface, of a drug active ingredient-containing film, wherein (1) the core particle includes a pharmaceutically acceptable inorganic material and a disintegration promoting agent, (2) the inorganic material is poorly soluble in water, (3) the inorganic material has a content of from 50 to 95 wt%, and (4) the core particle has a bulk density of at least 0.6 g/mL.

**Description**

TECHNICAL FIELD

The present invention relates to a core particle for a pharmaceutical preparation, which core particle itself disintegrates at a predetermined timing.

BACKGROUND ART

One known approach for manufacturing pharmaceutical preparations entails rendering core particles into a fluidized state and pouring therein either a drug (active ingredient) by itself or a mixture of the drug with a filler so as to coat the drug or mixture onto the surface of the core particles. In such a case, it is required of the core particles that (1) the core particles generally are spherical and of uniform particle size, and (2) the core particles do not break up (have a certain degree of physical strength) in the coating step.

To date, conventional core particles have been made using primarily organic materials. Examples include core particles composed solely of crystalline cellulose (Patent Document 1), core particles composed solely of sugar (Patent Document 2), core particles composed of sugar and crystalline cellulose (Patent Document 3), core particles composed of sugar and starch (Patent Document 4), and core particles which use one selected from the group consisting of sugar alcohols, vitamin C and sodium chloride (Patent Document 5). However, as already mentioned, it is a given that these core particles do not break up.

Patent Document 1: Japanese Patent Application Publication No. H7-173050
Patent Document 2: Japanese Patent Application Publication No. H6-205959
Patent Document 3: Japanese Patent No. 3219787
Patent Document 4: Japanese Patent Application Publication No. H9-175999
Patent Document 5: Japanese Patent No. 3447042

At the same time, with the recent advances being made in drug delivery systems technology, there exists a desire for pharmaceutical preparations which are capable of delivering a drug active ingredient at a desired rate and timing. Yet, core particles for pharmaceutical preparations are manufactured with the understanding that the particles themselves are not to break up, and so the notion of deliberately having core particles disintegrate has hitherto been unknown.

DISCLOSURE OF THE INVENTION

Therefore, a main object of this invention is to provide a core particle for a pharmaceutical preparation, which core particle makes it possible to deliver a relatively large amount of a drug active ingredient, in particular, at a predetermined timing.

In light of the problems with the existing art, the inventors have conducted extensive investigations. As a result, the inventors have discovered that the above object can be achieved by using core particles constituted in a certain way, and completed this invention.

Accordingly, the invention provides the following disintegratable core particle for a pharmaceutical preparation.

1. A disintegratable core particle for a pharmaceutical preparation, which core particle is adapted for formation, on a surface thereof, of a drug active ingredient-containing film, wherein

(1) the core particle includes a pharmaceutically acceptable inorganic material and a disintegration promoting ingredient,
(2) the inorganic material is poorly soluble in water,
(3) the inorganic material has a content of from 50 to 95 wt%, and
(4) the core particle has a bulk density of at least 0.6 g/mL.

2. The disintegratable core particle for a pharmaceutical preparation according to 1. above, wherein the core particle has a particle hardness of at least 200 g/mm$^2$.

3. The disintegratable core particle for a pharmaceutical preparation according to 1. above, wherein the core particle has a particle size distribution in which 5 wt% or less of the particles have a particle size below 45 $\mu$m, at least 90 wt% of the particles have a particle size of 45 $\mu$m or more but less than 500 $\mu$m, and 5 wt% or less of the particles have a particle size of 500 μm or more.

4. The disintegratable core particle for a pharmaceutical preparation according to 1. above, wherein the core particle has a particle size distribution in which 5 wt% or less of the particles have a particle size below 45 μm, at least 90 wt% of the particles have a particle size of 45 μm or more but less than 150 μm, and 5 wt% or less of the particles have a particle size of 150 μm or more.

5. The disintegratable core particle for a pharmaceutical preparation according to 1. above, wherein the inorganic material has a solubility in water at 20°C of 1 g/30 mL or less.

6. The disintegratable core particle for a pharmaceutical preparation according to 1. above, wherein the inorganic material is at least one from among magnesium oxide, magnesium hydroxide, magnesium carbonate, dibasic calcium phosphate, silicon dioxide, aluminum hydroxide, calcium silicate and aluminum silicate.

7. The disintegratable core particle for a pharmaceutical preparation according to 1. above, wherein the disintegration promoting agent is at least one from among crospovidone, methylcellulose, low-substituted hydroxypropylcellulose (L-HPC), hydroxypropylcellulose (HPC), hydroxypropylmethylcellulose (HPMC), carboxymethylcellulose calcium, carboxymethylcellulose sodium, starch, guar gum, gum arabic and polyvinyl alcohol.

8. The disintegratable core particle for a pharmaceutical preparation according to 1. above, wherein the core particles have an average particle size of at least 50 μm.

9. The disintegratable core particle for a pharmaceutical preparation according to 1. above, wherein the core particle is obtained by granulating a mixture comprising the inorganic material and the disintegration promoting ingredient.

10. A drug-containing particle including a drug active ingredient-containing film formed on the surface of the core particle for a drug preparation according to 1. above.

11. The drug-containing particle according to 10. above, wherein the film contains a drug active ingredient and a filler.

Advantages of the invention

Because the disintegratable core particle for a pharmaceutical preparation of the present invention includes an inorganic material and a disintegration promoting agent, the core particle itself disintegrates in vivo within a fixed period of time following administration, thus enabling the drug active ingredient included in the film on the surface thereof to be delivered in a relatively large amount. Also, by controlling parameters such as the time until such disintegration, the drug active ingredient can be effectively introduced into a given organ, tissue or the like, enabling, for example, a higher therapeutic effect to be achieved.

BRIEF DESCRIPTION OF THE DRAWINGS

FIG. 1 shows the results of scanning electron microscopic observation (400X) of a starting powder (magnesium carbonate powder) used in an example of the invention.

FIG. 2 shows the results of scanning electron microscopic observation (2,000X) of a starting powder (anhydrous dibasic calcium phosphate powder) used in an example of the invention.

FIG. 3 shows the results of scanning electron microscopic observation (2,000X) of a starting powder (magnesium oxide powder) used in an example of the invention.

FIG. 4 shows the results of scanning electron microscopic observation (500X) of a starting powder (L-HPC powder) used in an example of the invention.

FIG. 5 shows the results of scanning electron microscopic observation (1,000X) of a starting powder (crospovidone) used in an example of the invention.

FIG. 6 shows the results of scanning electron microscopic observation (130X and 800X) of the core particles obtained in Example 1.

FIG. 7 shows the results of scanning electron microscopic observation (150X and 850X) of the core particles obtained in Example 2.

FIG. 8 shows the results of scanning electron microscopic observation (350X and 1,100X) of the core particles obtained in Example 3.

FIG. 9 shows the results of scanning electron microscopic observation (350X and 950X) of the core particles obtained in Example 4.

FIG. 10 shows the results of scanning electron microscopic observation (150X and 750X) of the core particles obtained in Example 5.

FIG. 11 shows the results of scanning electron microscopic observation (50X and 500X) of the core particles obtained in Example 6.

FIG. 12 shows the results of scanning electron microscopic observation (50X and 600X) of the core particles obtained in Examples 7 to 10.

FIG. 13 shows the results of scanning electron microscopic observation (50X and 450X) of the core particles obtained in Examples 11 and 12.

FIG. 14 is a graph showing the results of a dissolution test on the pharmaceutical preparation obtained in Example 11.

FIG. 15 is a graph showing the results of a dissolution test on the pharmaceutical preparation obtained in Example 12.

BEST MODE FOR CARRYING OUT THE INVENTION

The disintegratable core particle of the present invention is disintegratable core particle for a pharmaceutical preparation, the core particle being used for forming a film containing a drug active ingredient on a surface of the core particle, wherein

(1) the core particle comprises a pharmaceutically acceptable inorganic material and a disintegration promoting agent,
(2) the inorganic material is poorly soluble in water,
(3) the inorganic material has a content of from 50 to 95 wt%, and
(4) the core particle has a bulk density of 0.6 g/mL or more.

1. Disintegratable Core Particle for Pharmaceutical Preparation

A pharmaceutically (pharmacologically) acceptable, poorly water-soluble inorganic material is used as the inorganic material included in the disintegratable core particle for pharmaceutical preparation of the invention (inventive core particle). Preferred use can be made of, in particular, an inorganic material having a solubility in water at 20°C of 1 g/30 mL or less, and especially 1 g/100 mL or less. In materials that are capable of dissolving in water, shape retention property sometimes decreases with the infiltration of water. By contrast, in this invention, by using an inorganic material which is poorly soluble in water, a stable shape retention property can be achieved.

The type of inorganic material is not subject to any particular limitation, so long as it is poorly soluble in water. A known or commercially available inorganic material used for pharmaceutical preparations can be employed as the inorganic material. Exemplary inorganic materials include, for example, at least one type of poorly water-soluble inorganic material from among anhydrides and hydrates of phosphates, silicates, oxides and hydroxides. Of these, preferred use can be made of at least one from among the following: magnesium oxide, magnesium hydroxide, magnesium carbonate, dibasic calcium phosphate, silicon dioxide, aluminum hydroxide, calcium silicate and aluminum silicate. By using these inorganic materials, core particles capable of maintaining a desired shape retention property and desired particle hardness until disintegration can be advantageously formed.

The content of inorganic material in the core particle of the present invention is not particularly limited, although it is desirable that it be generally at least 50 wt%, especially at least 70 wt%, and particularly from 80 to 95 wt%. By setting the amount of inorganic material within the above range, the particle is able to exhibit excellent shape retention property while yet including a disintegration promoting agent.

The disintegration promoting agent is not limited as long as it functions to give rise to dissolution, swelling or the like in the presence of water and causes the core particle to disintegrate. For example, a known or commercially available disintegrant may be used for this purpose. Use may be made of at least one from among the following: crospovidone, methylcellulose, low-substituted hydroxypropylcellulose (L-HPC), hydroxypropylcellulose (HPC), hydroxypropylmethylcellulose (HPMC), carboxymethylcellulose calcium, carboxymethylcellulose sodium, starch, guar gum, gum arabic and polyvinyl alcohol.

The content of the disintegration promoting agent in the inventive core particle of the present invention may be adjusted in such a way as to disintegrate at the desired site and time in the period following administration and up to elimination from the body, and may be suitably set in accordance with the type of disintegration promoting agent and the type of inorganic material used. It is desirable to set the content in the range of generally from about 5 to about 50 wt%, and preferably from 5 to 20 wt%.

In addition to the inorganic material and the disintegration promoting agent described above, known or commercially available pharmaceutical excipients which are not a drug active ingredient may be optionally included in the core particle of the present invention. That is, pharmaceutical excipients other than those used as the above inorganic material and the above disintegration promoting agent may be contained. For example, vehicles (e.g., lactose), binders (e.g., ethyl cellulose), lubricants (e.g., magnesium stearate, calcium stearate), and pH adjustors (e.g., citric acid, acetic acid, sulfuric acid, hydrochloric acid, lactic acid, sodium hydroxide, potassium hydroxide) may be used as such pharmaceutical excipients. The content of pharmaceutical excipients is not subject to any particular limitation so long as it is in a range that does not detract from the  advantageous effects of the invention, although it is desirable to set the content within a range of 20 wt% or less.

The shape of the inventive core particle is not particularly limited and may be, for example, spherical, cylindrical, flake or of non-uniform shape, although from the standpoint of flow properties and the like, a spherical shape is generally desirable.

The core particle of the present invention has a bulk density which is set to generally at least 0.6 g/mL. This makes it possible for an excellent core particle performance (e.g., excellent coatability) to be obtained. From this standpoint, in the present invention, it is especially preferable to set the bulk density of the core particle to from 0.7 to 1.0 g/mL.

The core particle of the present invention has a hardness which, although not particularly limited, is preferably at least 200 g/mm$^2$. By setting the particle hardness in this range, it is possible to more effectively prevent breakage and pulverization of the core particle in the coating step used to form a drug active ingredient-containing film on the surface of the core particle of the present invention. The upper limit value for hardness, although not subject to any particular limitation, may generally be set to about 3,000 g/mm$^2$.

The core particles of the present invention have an average particle size which, in general, can be suitably set in the range of at least 50 μm, preferably from 50 to 500 μm, and more preferably from 50 to 350 μm.

Also, it is desirable for the particle size distribution to be one in which 5 wt% or less of the particles have a particle diameter below 45 μm, at least 90 wt% of the particles have a particle diameter of 45 μm or more but less than 500 μm, and 5 wt% or less of the particles have a particle diameter of 500 μm or more.

Accordingly, in this invention, preferred use can be made of core particles having particle size distributions such as those in (A) to (C) below:

(A) core particles in which 5 wt% or less (preferably 2 wt% or less) of the particles have a particle diameter less than 45 μm, at least 90 wt% (preferably at least 96 wt%) of the particles have a particle diameter of 45 μm or more but less than 350 μm, and 5 wt% or less (preferably 2 wt% or less) of the particles have a particle diameter of 350 μm or more;

(B) core particles in which 5 wt% or less (preferably 2 wt% or less) of the particles have a particle diameter less than 45 μm, at least 90 wt% (preferably at least 96 wt%) of the particles have a particle diameter of 45 μm or more but less than 150 μm, and 5 wt% or less (preferably 2 wt% or less) of the particles have a particle diameter of 150 μm or more; and

(C) core particles in which 5 wt% or less (preferably 2 wt% or less) of the particles have a particle diameter less than 350 μm, at least 90 wt% (preferably at least 96 wt%) of the particles have a particle diameter of 350 μm or more but less than 500 μm, and 5 wt% or less (preferably 2 wt% or less) of the particles have a particle diameter of 500 μm or more.

The core particles of (A) and (B) above have relatively small particle sizes and, when used as tablets, a powder or the like, are able to provide a medicine which is smooth on the tongue and easy to swallow. Because it is difficult or impossible to form such fine core particles by using organic core particles, this is a distinguishing feature of the present invention which is dependent on the use of an inorganic material. The core particles of (C) above have a relatively large particle size and can be advantageously used as core particles in a medicine to be filled into capsules.

Setting the angle of repose in the core particle of the present invention to generally 40° or less, preferably 37° or less, and more preferably 35° or less, is preferred from the standpoint of, for example, uniformly pouring the core particles into and discharging them from equipment, and also from the standpoint of forming a uniform coating layer on the core particles.

The core particles of the present invention are preferably obtained by granulating a composition (starting powder as a starting material) containing the above-described inorganic material and disintegration promoting agent. In addition to the inorganic material and the disintegration promoting agent, where necessary, the starting powder may also contain the above-described pharmaceutical excipients. The method of granulation used is preferably one of the methods mentioned subsequently in Section 2.

The inventive core particles can be used by forming on the surfaces thereof a drug active ingredient-containing film. For example, specific drug-containing particles (pharmaceutical product) can be manufactured by coating the surfaces of the inventive core particles with a drug active ingredient-containing composition.

Examples of drug active ingredients include, but are not limited to, antihyperlipidemic drugs, antiulcerogenic drugs, antihypertensive drugs, antidepressant drugs, antiasthmatic drugs, antiepileptic drugs, antiallergy drugs, antibacterials, anticancer drugs, analgesics, anti-inflammatory drugs, diabetes drugs, antimetabolic drugs, osteoporosis drugs, antiplatelet drugs, antiemetic drugs, hormone drugs and anesthetics.

Moreover, where necessary, known or commercially available pharmaceutical excipients other than the above-described disintegration promoting agent may be contained in these compositions. As the pharmaceutical excipients, for example, vehicles, binders, lubricants, and pH adjustors can be used. The content of these pharmaceutical excipients may be suitably set according to, for example, the types of pharmaceutical additives and the content of the drug active ingredient.

No limitation is imposed on the method of coating the surfaces of the inventive core particles with a drug active ingredient-containing composition. For example, use may be made of known granulating methods such as mixing granulation, fluidized-bed granulation and tumbling granulation. Such granulation may be carried out using a known or commercially available granulator. The thickness of the drug active ingredient-containing film may be adjusted within the range of generally about from 1 to 100 μm.

2. Method of Producing Inventive Core Particles

The inventive core particles can be obtained by, for example, granulating a composition (starting powder) containing the above-described inorganic material and disintegration promoting agent.

A pharmaceutically acceptable inorganic material which is poorly soluble in water may be used as the starting powder. That is, a fine powder of any of the inorganic materials mentioned above may be used.

The average particle diameter of the starting powder can be suitably set according to, for example, the desired particle diameter of the core particles of the present invention, and may be set to generally from 0.1 to 40 μm, and especially from 0.1 to 20 μm.

No particular limitation is imposed on the method of granulation. Methods that may be used include, for example, tumbling granulation, mixing granulation, fluidized-bed granulation, compaction forming (compression granulation), film-forming treatment, magnetic property-based treatment, surface modification, sintering, vibration compacting, pressure swing granulation, vacuum forming and spray drying, as well as freeze drying and co-precipitation. Granulation may be carried out using a known or commercially available granulator. Of these methods of granulation, mixing granulation is especially suitable in the present invention.

Granulation may be carried out by either a wet method or a dry method, although granulation by a wet method is especially suitable in the present invention. When granulation is carried out by a wet method, no limitation is imposed on the type of solvent, although preferred use can be made of water or an aqueous solvent. Aqueous solvents that can be advantageously used include mixed solvents of ethanol and water (in a volumetric ratio of ethanol to water of about 1 : 0 to 1 : 5). The amount of solvent used may be set to generally about from 30 to 300 parts by weight per 100 parts by weight of the starting powder.

In one preferred method, granulation is carried out with a high-speed stirring-type mixing granulator by pouring the starting powder into the granulator and mixing the powder by the stirrers in the granulator while spraying it with a solvent to fluidize the powder. In such a high-speed type mixing granulator, when an agitator and a chopper are used as the stirrers, although the speed settings will depend also on other conditions, granulation can be advantageously carried out by setting the agitator speed to about from 500 to 1,000 rpm and the chopper speed to about from 1,000 to 1,500 rpm. The wet granulated material that has formed may be dried within the granulator (hopper), or the wet granulated material may be removed from the granulator (hopper) and dried. The core particles of the present invention can be obtained by subsequently carrying out classification to the target particle size distribution.

EXAMPLES

The features of the invention are explained more fully below by way of the following Examples and Comparative examples, although these examples do not limit the scope of the invention.

Examples 1 to 10 and Comparative Examples 1 to 3

Core particles were produced by charging the inorganic materials and pharmaceutical excipients (disintegrants, etc.) shown in Table 1 as the starting materials into a high-speed type mixing granulator ("LFS-GS-2J", from Fukae Powtec Co., Ltd.) so as to give the compositions shown in Table 2, adding water and wet granulating, then drying at 80°C for 24 hours. The granulation conditions in the respective Examples (EX) and Comparative examples (CE) are as shown in Table 2.

Table 1

| | | Characteristics | | | |
|---|---|---|---|---|---|
| | | Appearance | Average particle size ($\mu$m) | Bulk density (g/mL) | Angle of repose |
| Inorganic materials | Magnesium oxide powder (Tomita Pharmaceutic al Co., Ltd.) | white powder | 4.1 | 0.38 | 39° |
| | Anhydrous dibasic calcium phosphate powder (Tomita Pharmaceutic al Co., Ltd.) | white powder | 3.3 | 0.32 | 56° |
| | Magnesium carbonate powder (Tomita Pharmaceutic al Co., Ltd.) | white powder | 19.5 | 0.56 | 52° |
| Pharmaceutical excipients | HPMC (disintegrant) | white powder | 88.7 | 0.42 | 54° |
| | L-HPC (disintegrant) | white powder | 18.2 | 0.30 | 59° |
| | Crospovidone (disintegrant, wicking agent) | white powder | 5.9 | 0.19 | 63° |
| | Lactose (vehicle, wicking agent) | white powder | 46.5 | 0.63 | 51° |
| | D-Mannitol (vehicle, wicking agent) | white powder | 38.1 | 0.63 | 42° |

Table 2

| | | EX 1 | EX 2 | EX 3 | EX 4 | EX 5 | EX 6 | EX 7 | EX 8 | EX 9 | EX 10 | CE 1 | CE 2 | CE 3 |
|---|---|---|---|---|---|---|---|---|---|---|---|---|---|---|
| Starting materials | Magnesium oxide powder | 240 g (80%) | 240 g (80%) | -- | -- | -- | -- | 150 g (50%) | -- | -- | -- | 300 g (100%) | -- | -- |
| | Anhydrous dibasic calcium phosphate powder | -- | -- | 240 g (80%) | 270 g (90%) | 270 g (90%) | | -- | -- | -- | -- | -- | 300 g (100%) | -- |
| | Magnesium carbonate powder | -- | -- | -- | -- | -- | 270 g (90%) | | 270 g (90%) | 210 g (70%) | 150 g (50%) | -- | -- | 300 g (100%) |
| | HPMC | -- | 30 g (10%) | -- | 15 g (5%) | 30 g (10%) | 15 g (5%) | -- | -- | -- | -- | -- | -- | -- |
| | L-HPC | 15 g (5%) | 30 g (10%) | -- | 15 g (5%) | -- | 15 g (5%) | 150 g (50%) | 30 g (10%) | 90 g (30%) | 150 g (50%) | -- | -- | -- |
| | Crospovidone | -- | -- | 30 g (10%) | -- | -- | -- | -- | -- | -- | -- | -- | -- | -- |
| | Lactose | 45 g (15%) | -- | -- | -- | -- | -- | -- | -- | -- | -- | -- | -- | -- |
| | D-Mannitol | -- | -- | 30 g (10%) | -- | -- | -- | -- | -- | -- | -- | -- | -- | -- |
| Granulating conditions | Solvent | ethanol | ethanol | ethanol : water = 1 : 1 | | | | ethanol | ethanol | ethanol | ethanol | ethanol | water | water |
| | Amount of solvent added (mL) | 180 | 190 | 140 | 170 | 130 | 160 | 190 | 215 | 240 | 265 | 170 | 120 | 110 |
| | Agitator speed (rpm) | 800 | 800 | 800 | 800 | 800 | 800 | 800 | 800 | 800 | 800 | 800 | 800 | 800 |
| | Chopper speed (rpm) | 1,500 | 1,500 | 1,500 | 1,500 | 1,500 | 1,500 | 1,500 | 1,500 | 1,500 | 1,500 | 1,500 | 1,500 | 1,500 |
| | Granulation time (sec) | 900 | 930 | 720 | 660 | 660 | 780 | 1,820 | 2,880 | 3,720 | 3,960 | 840 | 870 | 780 |
| Core particle properties | Appearance | spherical | spherical | spherical | spherical | spherical | spherical | spherical | spherical | spherical | spherical | spherical | spherical | spherical |
| | Particle hardness (g/mm$^2$) | 410 | 523 | 215 | 230 | 315 | 458 | 417 | 1,346 | 745 | 634 | 980 | 675 | 2,120 |
| | Bulk density (g/mL) | 0.77 | 0.83 | 0.67 | 0.71 | 0.71 | 0.63 | 0.71 | 0.72 | 0.72 | 0.68 | 1.00 | 0.77 | 0.83 |
| | Angle of repose | 28° | 30° | 37° | 35° | 33° | 33° | 38° | 35° | 37° | 37° | 28° | 31° | 31° |
| | Particle size distribution (%) ≥150 μm | 0.2 | 0.1 | 0.3 | 0.1 | 0.0 | 0.3 | 0.2 | 0.1 | 0.5 | 0.4 | 0.1 | 0.0 | 0.2 |
| | 45 to 150 μm | 96.7 | 96.3 | 97.2 | 95.6 | 95.9 | 96.9 | 96.4 | 98.1 | 96.7 | 97.4 | 98.7 | 96.1 | 97.4 |
| | ≤45 μm | 3.1 | 3.6 | 2.5 | 4.5 | 4.1 | 2.8 | 3.4 | 1.8 | 2.8 | 2.2 | 1.2 | 3.9 | 2.4 |

Test Example 1

The appearance (shape), hardness, bulk density, angle of repose and particle size distribution of the core particles obtained in the respective Examples of the invention and Comparative examples were measured. The results are shown in Table 2. The following methods were used to measure these properties.

(1) Appearance (Shape)

The particles were examined with a scanning electron microscope.

(2) Particle Hardness

Using a particle hardness tester ("Grano", from Okada Seiko Co., Ltd.), the peak value (g) in the crushing strength of a single particle was measured, and the average value for 20 particles was determined.

(3) Bulk Density

Twenty grams of sample was placed in a 50 mL graduated cylinder, following which the cylinder was set in a tapped density powder tester ("TMP-7-P", available from Tsutsui Rikagaku Kikai Co., Ltd.), and testing was carried out under the following measurement conditions: number of taps:100, tapping height:4 cm, and tapping speed:36 taps/min. After testing, the volume F (mL) was visually measured. The bulk density (g/mL) was then calculated as 20/F.

(4) Angle of Repose

A hopper was placed at a position 100 mm above a 50 mm diameter dish and the sample was dropped, a small amount at a time, from the hopper onto the dish, creating a conical mound of the sample. The height (h) of this mound when the sample stopped sliding and came to rest was measured, and the angle that forms between the dish and the slope of the mound (angle of repose $\alpha = \tan^{-1}(h/25 \text{ mm})$) was calculated.

(5) Particle Size Distribution

The sample was ultrasonically agitated (frequency, 400 Hz), then dispersed in acetone, and measurement was carried out by laser diffractometry in an acetone solvent. The instrument used for measurement was the "MICROTRAC HRA Model No. 9320-X100", from Honeywell.

Test Example 2

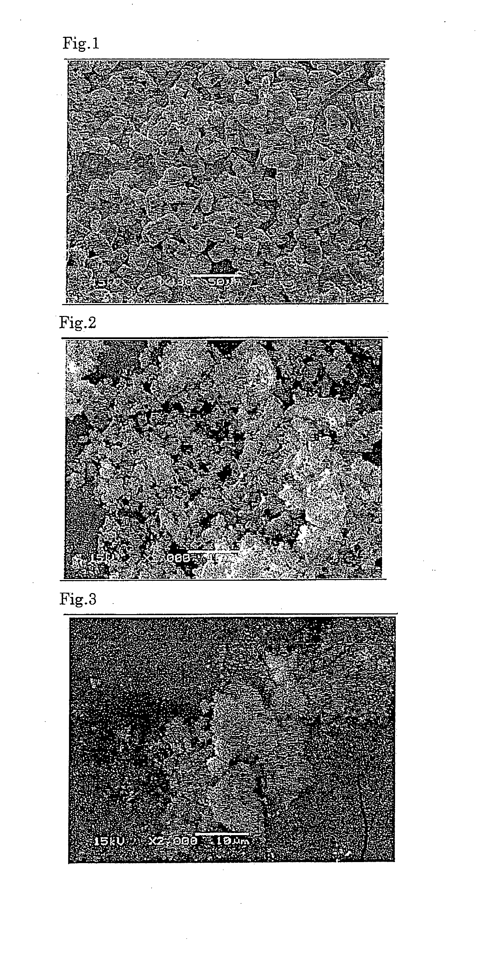

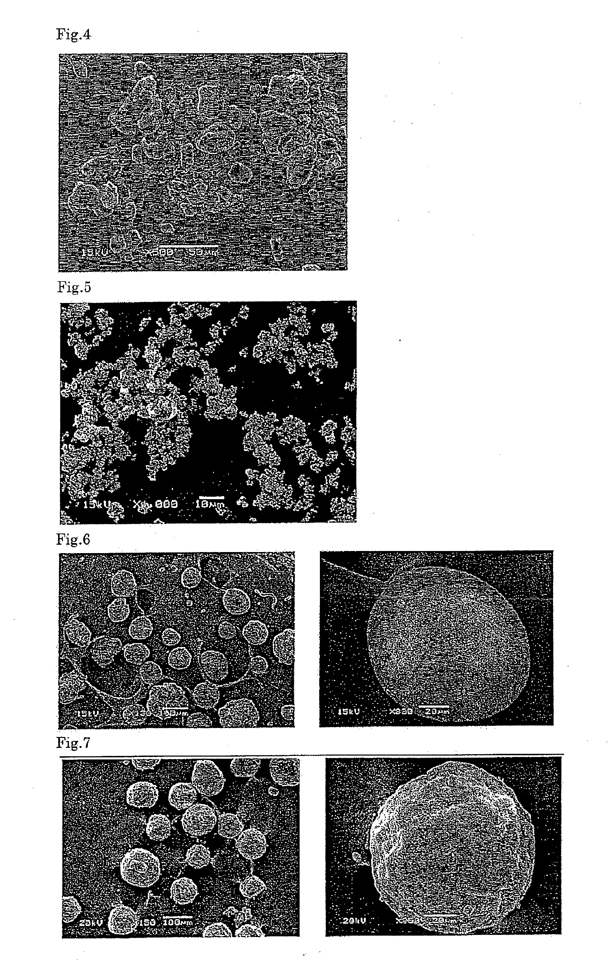

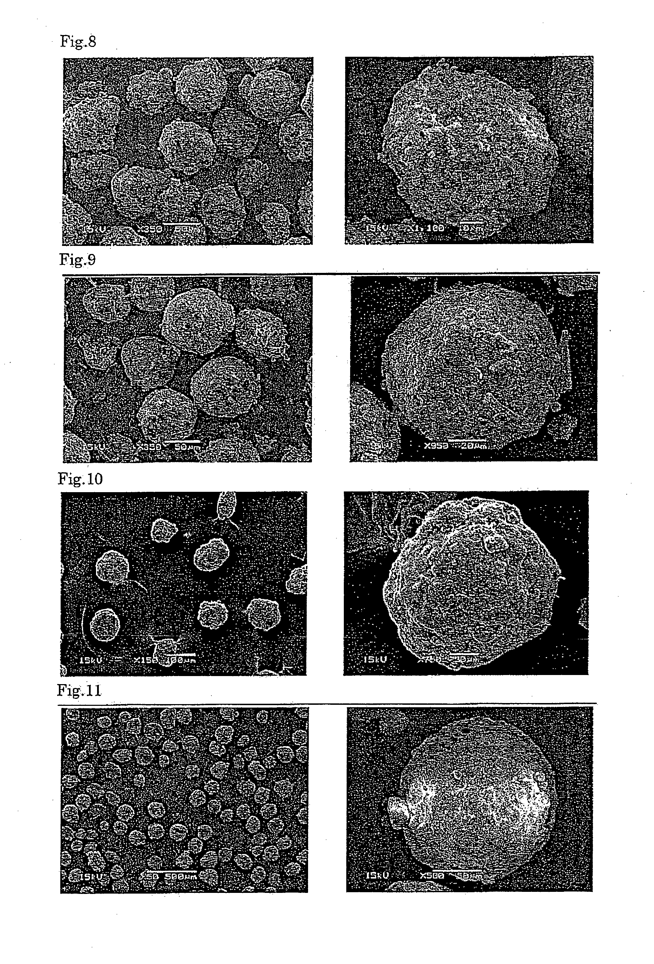

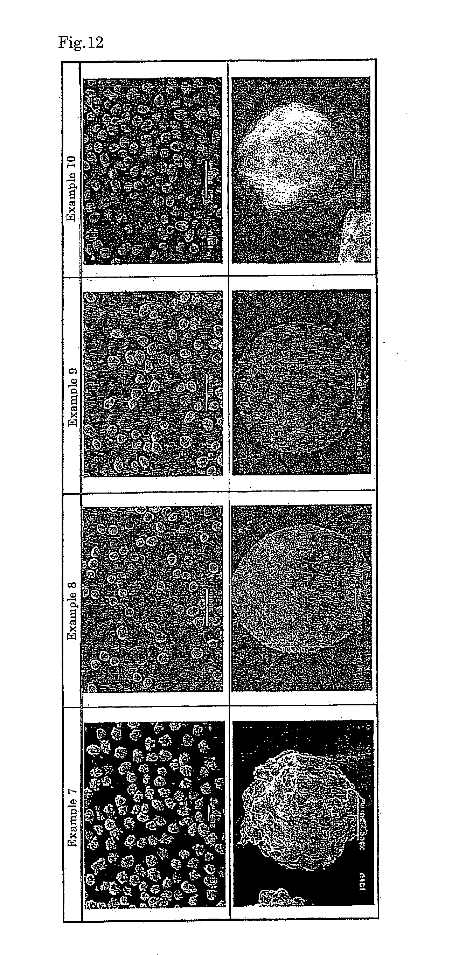

The starting powders used in Examples and Comparative examples were examined with a scanning electron microscope. The results are shown in FIGS. 1 to 5. In addition, the inventive core particles obtained in Examples 1 to 10 using these starting powders were examined with a scanning electron microscope. These latter results are shown in FIGS. 6 to 12. As is apparent from these results, substantially spherical particles can be produced using starting materials composed of particles having uneven shape.

Test Example 3

The disintegratability of the core particles obtained in Examples of the invention and Comparative examples were examined. The test method was as follows. First, 1 g of core particles was placed in a 200 mL Erlenmeyer flask, and water was added up to the 50 mL line. The flask was then shaken on a shaker for 10 minutes at 37°C and 100 rpm. The average particle diameters (D50) after 1 minute, 3 minutes, 5 minutes and 10 minutes of shaking were measured, and the change over time in the degree of particle disintegration was thereby determined. Those results are shown in Table 3.
Table 3

| Shaking time | Average particle diameter (D = 50) | | | | | | | | | | | | |
|---|---|---|---|---|---|---|---|---|---|---|---|---|---|
| | EX 1 | EX 2 | EX 3 | EX 4 | EX 5 | EX 6 | EX 7 | EX 8 | EX 9 | EX 10 | CE 1 | CE 2 | CE 3 |
| 0 min | 106 μm | 111 μm | 101 μm | 104 μm | 103 μm | 108 μm | 120 μm | 118 μm | 111 μm | 105 μm | 113 μm | 92 μm | 105 μm |
| 1 min | 87 μm | 98 μm | 52 μm | 32 μm | 37 μm | 7 6 μm | 42 μm | 57 μm | 27 μm | 2 3 μm | 114 μm | 84 μm | 103 μm |
| 3 min | 52 μm | 82 μm | 11 μm | 7 μm | 9 μm | 52 μm | 34 μm | 54 μm | 28 μm | 19 μm | 109 μm | 87 μm | 107 μm |
| 5 min | 42 μm | 63 μm | 7 μm | 7 μm | 6 μm | 45 μm | 31 μm | 56 μm | 2 6 μm | 20 μm | 110 μm | 81 μm | 100 μm |
| 10 min | 37 μm | 51 μm | 6 μm | 6 μm | 6 μm | 41 μm | 3 2 μm | 51 μm | 2 6 μm | 2 0 μm | 111 μm | 83 μm | 103 μm |

As shown in Table 3, the particle diameter remained substantially unchanged in Comparative examples, whereas the average particle diameter (D50) fell to one-half of the initial value after 3 minutes in Example 1, after 10 minutes in Example 2, after 1 minute in Example 3, within 1 minute in Example 4, within 1 minute in Example 5, after 3 minutes in Example 6, within 1 minute in Example 7, within 3 minutes in Example 8, within 1 minute in Example 9, and within 1 minute in Example 10. It is thus apparent that, in this invention, not only can the core particles be disintegrated, the time until disintegration can also be controlled.

Examples 11 and 12

Pharmaceutical preparations composed of two types of drug-containing particles were produced by using the core particles obtained in Example 7 and carrying out coating treatment on the surfaces of the core particles. The ingredients shown in Table 4 were used in the indicated amounts per 200 g of the core particles. Table 4 also shows the coating treatment conditions.
Table 4

| | Example 11 | Example 12 |
|---|---|---|
| Apparatus used | MP-01SFP (Powrex) | |
| Unit used | SPC (Wurster) | |

(continued)

|  | Example 11 | Example 12 |
|---|---|---|
| Spray liquid rate | 2.5 g/min | 5 g/min |
| Inlet temperature | 50°C | 60°C |
| Outlet temperature | 40°C | 50°C |
| Flow rate | 45 m$^3$/h | 45 m$^3$/h |
| Spray air pressure | 0.3 MPa | 0.3 MPa |
| Spray air flow rate | 30 L/min | 30 L/min |
| Example 7 | 200 g | 200 g |
| Ibuprofen | 20 g | -- |
| Rabeprazole Na | -- | 20 g |
| HPMC (TC-5) | 10 g | 10 g |
| Ultrapure water | 10 g | 10 g |
| Ethanol (99.5) | 190 g | 190 g |

Test Example 4

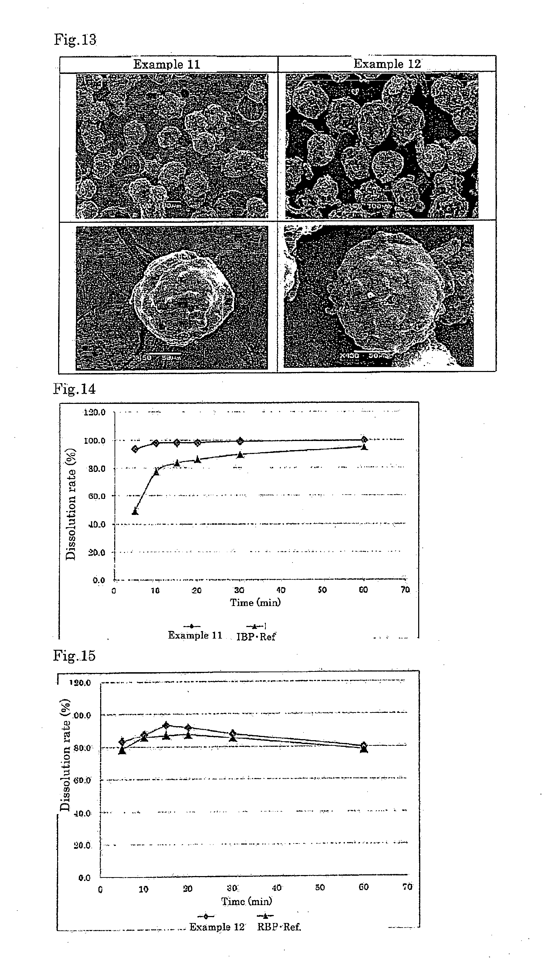

The particle appearance (shape), particle hardness, angle of repose and particle size distribution for the pharmaceutical preparations composed of the drug-containing particles obtained in Examples 11 and 12 were investigated by the same methods as in Test Example 1. The results are shown in Table 5. In addition, FIG. 13 shows the results of observations on the particle appearance (shape).

Table 5

|  |  | Example 11 | Example 12 |
|---|---|---|---|
| Angle of repose | | 54.2° | 52.8° |
| Particle hardness | | 151 g/mm$^2$ | 636 g/mm$^2$ |
| Particle size distribution | 22M on (710 $\mu$m) | 0.4% | 0.8% |
|  | 30M on (500 $\mu$m) | 5.0% | 2.7% |
|  | 42M on (355 $\mu$m) | 22.6% | 15.7% |
|  | 60M on (250 $\mu$m) | 29.3% | 29.8% |
|  | 83M on (180 $\mu$m) | 18.40% | 21.0% |
|  | 100M on (150 $\mu$m) | 10.9% | 11.3% |
|  | 140M on (106 $\mu$m) | 11.7% | 15.9% |
|  | 200M on (75 $\mu$m) | 1.4% | 1.3% |
|  | 200M pass | 0.3% | 1.5% |

Test Example 5

Dissolution tests were carried out, according to the procedures shown in Tables 6 to 8 and described below, on pharmaceutical preparations composed of the drug-containing particles obtained in Examples 11 and 12. The results are shown in Tables 9 and 10 and in FIGS. 14 and 15.

Ibuprofen (Example 11)

(1) Measurement of Ibuprofen Content

The following method is used to measure the ibuprofen content. First, 115 mg of the core particles is precisely weighed out and placed in a graduated cylinder containing about 80 mL of the second fluid specified in the Japanese Pharmacopoeia (JP second fluid), then the volume is brought up to 100 mL with the JP second fluid. Next, at least 20 mL of the resulting solution is collected and filtered with a membrane filter having a pore size of 0.45 $\mu$m or less. The first 10 mL of filtrate is discarded, and the remaining filtrate is used as the sample solution. In a separate procedure, 25 mg of ibuprofen (reference) is precisely weighed out, then dissolved in acetonitrile and brought up to exactly 50 mL. Five mL of this solution is then precisely measured out and brought up to exactly 25 mL by adding JP second fluid, thereby giving a reference solution (about 100 ppm). Next, 50 $\mu$L each of the sample solution and the reference solution are precisely measured out, the ibuprofen peak areas AT and As of the respective solutions are measured in accordance with "2.01 Liquid Chromatography" under "General Tests" in the Japanese Pharmacopoeia (JP), and the ibuprofen content is calculated from the following formula:

$$\text{Ibuprofen content (wt\%)} = WS \times 5/50 \times 1/25 \times (AT/AS) \times 10/C$$

where

WS: amount of ibuprofen reference weighed out (mg); and

C: amount of ibuprofen weighted out (g).

The test conditions in the above-mentioned liquid chromatography are as shown in Table 6.
Table 6

| Detector | Ultraviolet absorptiometer (measurement wavelength, 246 nm) |
|---|---|
| Column | Stainless steel tube (inside diameter, 4.6 mm; length, 15 cm) packed with octadecylsilylated silica gel (average particle size, 5 $\mu$m) for liquid chromatography (ODS-3) |
| Column temperature | Fixed temperature near 40°C (40°C) |
| Mobile phase | Acetonitrile/0.05 mol/L Monobasic sodium phosphate test solution (pH 2.6) mixture (volume ratio, 3 : 2)<br><br>The monobasic sodium phosphate test solution is prepared by dissolving 6 g of monobasic sodium phosphate in about 800 mL of ultrapure water, adjusting the pH to 2.6 with dilute hydrochloric acid, and adding water to exactly 1,000 mL. |
| Flow rate | Adjusted so that the ibuprofen retention time is about 6 minutes. |

(2) Dissolution Test

The liquid sampled in the dissolution test is used directly as the sample solution. Analysis according to "2.01 Liquid Chromatography" is carried out on this sample solution. Testing of the ibuprofen content in the sample solution is carried out under the conditions indicated in (1) above.
In a separate operation, 25 mg of ibuprofen reference is precisely weighed out and dissolved in acetonitrile while bringing the volume up to exactly 50 mL. Next, 5 mL of this solution is precisely measured out and JP second fluid is added so as to bring the volume up to exactly 25 mL, thereby giving a reference solution (about 100 ppm). In a separate operation, 5 mL of this solution is precisely measured out and JP second fluid is added so as to bring the volume up to 50 mL, thereby giving a 50 ppm reference solution. Next, 10 mL of this solution is precisely measured out and JP second fluid is added so as to bring the volume up to exactly 25 mL, thereby giving a 200 ppm reference solution. Liquid chromatography is carried out on these reference solutions, and the slopes (a) and intercepts (t) of the straight lines obtained by plotting the peak areas and concentrations of the ibuprofen references are determined. The dissolution rate is determined by entering the content B in the sample solution into the following formula:

```
Dissolution rate (%) = {(Qt - t)/a × 900/1,000}/(C × B/100) ×

                                  100
```

where

Qt:   peak area of sample solution;
C:    amount of ibuprofen measured out (mg); and
B:    ibuprofen content of coated product (%).

Rabeprazole (Example 12)

(1) Measurement of Rabeprazole Content

The following method is used to measure the rabeprazole content. First, 20 mg of the core particles is precisely weighed out and dissolved by adding 30 mL of an aqueous NaOH solution (0.5 M), 45 mL of methanol is added, then the volume is brought up to exactly 100 mL with a water-methanol solution (2 : 3). Two mL of this solution is precisely measured out and brought up to exactly 20 mL with a water-methanol solution (2 : 3), thereby giving a reference solution. Liquid chromatography is carried out on this reference solution, and the peak area (Qs) of rabeprazole is determined (Note: The linearity verification range is 4 ppm.). In a separate procedure, 230 mg of sample is precisely weighed out and dissolved by adding 30 mL of an aqueous NaOH solution (0.5 M), 45 mL of methanol is added, then the volume is brought up to exactly 100 mL with a water-methanol solution (2 : 3). This solution is centrifuged for 10 minutes at 3,000 rpm, then 2 mL of the supernatant is precisely measured out and a water-methanol solution (2 : 3) is added to a volume of exactly 20 mL, thereby giving a sample solution. The peak area (Qt) of rabeprazole is determined for the test solution by carrying  out liquid chromatography.

```
Content (%) = (Qt)/(Qs) × 20 × 10 × 100/1,000/sampling amount

of rabeprazole Na (mg) × 100
```

The test conditions in the above-mentioned liquid chromatography are as shown in Table 7.
Table 7

| <Assay Conditions> n = 3 Calibration Curve Method | |
| --- | --- |
| Detector | Ultraviolet absorptiometer (measurement wavelength, 290 nm) |
| Column | ODS (from GL Science): inside diameter, 4.6 mm; length, 15 cm |
| Column temperature | room temperature |
| Flow rate | 1.5 mL/min |
| Measurement time | 6 min |
| Mobile phase | methanol/50 mM phosphate buffer (pH 7.0) = 3 : 2 |
| Equipment | Shimadzu liquid chromatography |
| Analysis parameters | Atten = 5; slope = 500; Min. area = 200 |

(2) Dissolution Test

First, 20 mg of rabeprazole sodium is precisely weighed  out and dissolved by adding 30 mL of an aqueous NaOH solution (0.5 M), after which 45 mL of methanol is added, then the volume is brought up to exactly 100 mL with a water-methanol solution (2 : 3). Two mL of this solution is precisely measured out and brought up to exactly 100 mL with a water-methanol solution (2 : 3), thereby giving a 4 ppm reference solution. In a separate procedure, 2 mL of the same solution is precisely measured out and brought up to a volume of exactly 200 mL with a water-methanol solution (2 : 3), thereby giving a 2 ppm reference solution. Liquid chromatography is carried on these reference solutions, and the slopes (a) and intercepts (t) of the straight lines obtained by plotting the peak areas and concentrations of rabeprazole are determined.

One milliliter of an aqueous NaOH solution (0.5 M) is added to 5 mL of the sample solution at each sampling time, and the resulting solution is thoroughly mixed. Next, 4 mL of this solution is precisely measured out, then 2 mL of an aqueous NaOH solution (0.5 M) and 8 mL of methanol are added, following which the volume is brought up to exactly 20 mL with a water-methanol solution (2 : 3). Liquid chromatography is then carried out under the conditions described above, and the peak area (Qt) for rabeprazole is determined. Both the reference solutions and the sample solution are used as injection solutions after filtration with a membrane filter having a pore size of 0.2 $\mu$m.

Dissolution rate (%) = (Qt - t)/a x 5 x 6/5 x 900/1,000/amount of rabeprazole Na collected (mg) x 100

Table 8

|  | Example 11 | Example 12 |
|---|---|---|
| Amount of drug active ingredient charged | 115 mg/vessel | 230 mg/vessel |
| Apparatus | SR8-PLUS-8S (Hanson Research, USA) | SR8-PLUS-8S (Hanson Research, USA) |
| Method | paddle method | paddle method |
| Amount of test solution | 900 mL | 900 mL |
| Temperature of test solution | 37°C $\pm$ 0.5°C | 37°C $\pm$ 0.5°C |
| Rotational speed | 75 rpm | 50 rpm |
| Number of vessels | 3 | 3 |
| Test solution | JP second fluid (pH 6.8) | tris(hydroxymethyl)am inomethane buffer (pH 8.0) |
| Sampling times | 5 min, 10 min, 15 min, 30 min, 60 min | 5 min, 10 min, 15 min, 30 min, 60 min |

Table 9

| Dissolution time (min) | Dissolution rate (%) | |
|---|---|---|
|  | Example 11 | IBP - Ref. |
| 5 | 93.9 | 49.5 |
| 10 | 97.9 | 77.3 |
| 15 | 98.2 | 83.4 |
| 20 | 98.2 | 86.0 |
| 30 | 99.1 | 89.6 |
| 60 | 99.7 | 95.0 |

Table 10

| Dissolution time (min) | Dissolution rate (%) | |
|---|---|---|
| | Example 12 | RBP - Ref. |
| 5 | 83.3 | 78.6 |
| 10 | 87.3 | 86.0 |
| 15 | 93.2 | 87.1 |
| 20 | 91.8 | 87.5 |
| 30 | 87.7 | 85.5 |
| 60 | 79.9 | 78.4 |

As is apparent also from the results in Tables 9 and 10 and in FIGS. 14 and 15, dissolution of the drug active ingredients is more rapid in the case of the pharmaceutical preparations in Examples 11 and 12 than for the drug active ingredients alone.

**Claims**

1. A disintegratable core particle for a pharmaceutical preparation, the core particle being used for forming a film containing a drug active ingredient on a surface of the core particle, wherein

   (1) the core particle comprises a pharmaceutically acceptable inorganic material and a disintegration promoting agent,
   (2) the inorganic material is poorly soluble in water,
   (3) the inorganic material has a content of from 50 to 95 wt%, and
   (4) the core particle has a bulk density of 0.6 g/mL or more.

2. The disintegratable core particle for a pharmaceutical preparation according to claim 1, wherein the core particle has a particle hardness of 200 $g/mm^2$ or more.

3. The disintegratable core particle for a pharmaceutical preparation according to claim 1, having a particle size distribution of no more than 5 wt% of the particles having a diameter less than 45 $\mu$m, at least 90 wt% of the particles having a diameter at least 45 $\mu$m but less than 500 $\mu$m, and no more than 5 wt% of the particles having a diameter at least 500 $\mu$m.

4. The disintegratable core particle for a pharmaceutical preparation according to claim 1, having a particle size distribution of no more than 5 wt% of the particles having a diameter less than 45 $\mu$m, at least 90 wt% of the particles having a diameter at least 45 $\mu$m but less than 150 $\mu$m, and no more than 5 wt% of the particles having a diameter at least 150 $\mu$m.

5. The disintegratable core particle for a pharmaceutical preparation according to claim 1, wherein the inorganic material has a solubility in water at 20°C of 1 g/30 mL or less.

6. The disintegratable core particle for a pharmaceutical preparation according to claim 1, wherein the inorganic material is at least one from among magnesium oxide, magnesium hydroxide, magnesium carbonate, dibasic calcium phosphate, silicon dioxide, aluminum hydroxide, calcium silicate and aluminum silicate.

7. The disintegratable core particle for a pharmaceutical preparation according to claim 1, wherein the disintegration promoting agent is at least one from among crospovidone, methylcellulose, low-substituted hydroxypropylcellulose (L-HPC), hydroxypropylcellulose (HPC), hydroxypropylmethylcellulose (HPMC), carboxymethylcellulose calcium, carboxymethylcellulose sodium, starch, guar gum, gum arabic and polyvinyl alcohol.

8. The disintegratable core particle for a pharmaceutical preparation according to claim 1, wherein the particles have an average particle diameter of 50 $\mu$m or more.

9. The disintegratable core particle for a pharmaceutical preparation according to claim 1, wherein the core particle is obtained by granulating a composition containing the inorganic material and the disintegration promoting agent.

10. A drug-containing particle comprising a drug active ingredient-containing film formed on the surface of the core particle for a drug preparation according to claim 1.

11. The drug-containing particle according to claim 10, wherein the film contains a drug active ingredient and a vehicle.

Fig.1

Fig.2

Fig.3

Fig.4

Fig.5

Fig.6

Fig.7

Fig.8

Fig.9

Fig.10

Fig.11

Fig.12

Fig.13

| Example 11 | Example 12 |
|---|---|
| | |
| | |

Fig.14

Example 11    IBP·Ref

Fig.15

Example 12    RBP·Ref.

**INTERNATIONAL SEARCH REPORT**

| International application No. |
| --- |
| PCT/JP2011/080276 |

A. CLASSIFICATION OF SUBJECT MATTER
*A61K9/14*(2006.01)i, *A61K47/02*(2006.01)i, *A61K47/04*(2006.01)i, *A61K47/26*
(2006.01)i, *A61K47/32*(2006.01)i, *A61K47/36*(2006.01)i, *A61K47/38*(2006.01)i

According to International Patent Classification (IPC) or to both national classification and IPC

B. FIELDS SEARCHED

Minimum documentation searched (classification system followed by classification symbols)
A61K9/14, A61K47/02, A61K47/04, A61K47/26, A61K47/32, A61K47/36,
A61K47/38

Documentation searched other than minimum documentation to the extent that such documents are included in the fields searched
Jitsuyo Shinan Koho          1922-1996    Jitsuyo Shinan Toroku Koho    1996-2012
Kokai Jitsuyo Shinan Koho    1971-2012    Toroku Jitsuyo Shinan Koho    1994-2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| P,X | JP 2011-26307 A  (Tomita Pharmaceutical Co., Ltd.), 10 February 2011 (10.02.2011), claims 1 to 11; examples 3, 4 (Family: none) | 1-11 |
| P,X | JP 2011-157348 A  (Fuji Chemical Industry Co., Ltd.), 18 August 2011 (18.08.2011), claims 1 to 9; examples 1 to 4 (Family: none) | 1-11 |
| X,Y | JP 5-92918 A  (Takeda Chemical Industries, Ltd.), 16 April 1993 (16.04.1993), claim 1; paragraphs [0018], [0033]; example 14 (Family: none) | 1-11 |

☒  Further documents are listed in the continuation of Box C.          ☐  See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| Date of the actual completion of the international search <br> 25 January, 2012 (25.01.12) | Date of mailing of the international search report <br> 07 February, 2012 (07.02.12) |
| Name and mailing address of the ISA/ <br> Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2011/080276

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | WO 2009/157214 A1  (Fuji Chemical Industry Co.,<br>Ltd.),<br>30 December 2009 (30.12.2009),<br>claims 1 to 7; example 3<br>(Family: none) | 1-5,7-11<br>1-11 |
| X | JP 2006-16329 A  (Lion Corp.),<br>19 January 2006 (19.01.2006),<br>paragraph [0024]<br>(Family: none) | 1-9 |
| X | JP 2007-15982 A  (Amato Pharmaceutical Products<br>Ltd.),<br>25 January 2007 (25.01.2007),<br>paragraph [0020]; example 1<br>(Family: none) | 1-9 |
| X | WO 00/71097 A1  (Takeda Chemical Industries,<br>Ltd.),<br>30 November 2000 (30.11.2000),<br>example 1<br>& AU 4778500 A | 1-9 |
| Y | WO 2009/72334 A1  (Tomita Pharmaceutical Co.,<br>Ltd.),<br>11 June 2009 (11.06.2009),<br>claims 1 to 17; paragraphs [0019], [0029]<br>& US 2010/0260856 A1     & EP 2223702 A1 | 1-11 |
| Y | JP 2010-6769 A  (Tomita Pharmaceutical Co.,<br>Ltd.),<br>14 January 2010 (14.01.2010),<br>claims 1 to 9; paragraphs [0023], [0028]<br>(Family: none) | 1-11 |

Form PCT/ISA/210 (continuation of second sheet) (July 2009)

**REFERENCES CITED IN THE DESCRIPTION**

**Patent documents cited in the description**

- JP H7173050 B
- JP H6205959 B
- JP 3219787 B
- JP H9175999 B
- JP 3447042 B